# EUROPEAN PATENT SPECIFICATION

(11) **EP 4 608 342 B1**
(45) Date of publication and mention of the grant of the patent: **01.07.2026**
(21) Application number: 23754952.2
(22) Date of filing: 18.07.2023
(51) Int. Cl.: A61F 5/00

(54) **DYNAMIC ANCHORING OF SMALL INTESTINAL LINERS**
DYNAMISCHE VERANKERUNG VON DÜNNDARMLINERN
ANCRAGE DYNAMIQUE DE GAINES DE L'INTESTIN GRÊLE

(30) Priority: 27.10.2022 US 202263420049 P
(43) Date of publication of application: 03.09.2025
(73) Proprietor: Boston Scientific Scimed Inc., Maple Grove, Minnesota 55311 (US); Mayo Foundation for Medical Education and Research, Rochester, Minnesota 55905 (US)
(72) Inventor: MCGOWAN, Roger W., Otsego, Minnesota 55362 (US); ABU DAYYEH, Barham K., Rochester, Minnesota 55902 (US); WIEDMANN, Alexander James, Columbia Heights, Minnesota 55421 (US); KRAUTKREMER, Daniel Lee, Plymouth, Minnesota 55441 (US); BRAND, Jessica, Maple Grove, Minnesota 55369 (US)
(74) Representative: Vossius & Partner Patentanwälte Rechtsanwälte mbB
(86) International application number: PCT/US2023/070418
(87) International publication number: WO 2024/091720

(56) References cited:
- KR-A- 20110 059 190
- US-A1- 2012 184 893
- US-A1- 2014 276 338
- US-A1- 2021 015 646
- US-A1- 2021 128 334

## Description

### Cross-Reference To Related Applications

This application claims the benefit of priority of U.S. Provisional Application No. 63/420,049, filed October 27, 2022.

### Technical Field

The present disclosure pertains to medical devices such as gastric bypass devices. More particularly, the present disclosure pertains to gastric bypass devices that include small intestinal liners.

### Background

A wide variety of intracorporeal medical devices have been developed for medical use, for example, surgical and/or intravascular use. Some of these devices include guidewires, catheters, medical device delivery systems (e.g., for stents, grafts, replacement valves, etc.), and the like. These devices are manufactured by any one of a variety of different manufacturing methods and may be used according to any one of a variety of methods.
US 2012/0184893 A1 discloses a gastrointestinal device for implanting within a pylorus, a duodenal bulb, and a duodenum of a patient's gastrointestinal tract includes an expandable structure including a proximal portion having a plurality of spring arms and a distal portion having a plurality of spring arms, the proximal and distal portions coupled by a rigid central cylinder having a diameter capable of fitting within the pylorus and having a length greater than a width of the pylorus. An intestinal bypass sleeve is coupled to at least one of the proximal and distal portions of the expandable structure and having a length sufficient to extend at least partially into the duodenum. In the expanded configuration, the proximal portion has a diameter larger than a maximum opening diameter of the pylorus and further wherein, in the expanded configuration, the distal portion has a diameter larger than a maximum opening diameter of the pylorus.
There is an ongoing need to provide alternative medical devices as well as alternative methods for manufacturing and/or using medical devices.

### Summary

The invention is defined by the features of the independent claim. The disclosure pertains to medical devices such as gastric bypass devices and more particularly to gastric bypass devices that include small intestinal liners. An example may be found in an implantable medical device. The implantable medical device includes an occlusion device adapted to be disposed within a patient's stomach relative to the patient's pylorus in order to prevent stomach contents from flowing around the occlusion device, the occlusion device including a distal outflow end. A liner extends distally from the occlusion device, the liner defining a lumen extending through the liner, the liner having a proximal end fluidly coupled with the distal outflow end and a distal end adapted to be disposed within the patient's jejunum such that stomach contents entering the occlusion device flow through the liner and exit through the distal end. A tether is secured relative to the occlusion device and adapted to limit proximal movement of the occlusion device.

Alternatively or additionally, the tether may be adapted to extend within the lumen of the liner.

Alternatively or additionally, the tether may be adapted to extend outside of the liner.

According to the invention, the tether includes a proximal end by which the tether is securable relative to the occlusion device and a distal end by which the tether is securable to an anchor.

Alternatively or additionally, the anchor may be adapted to be secured in place relative to the patient's small intestine or stomach wall.

Alternatively or additionally, the anchor may be adapted to pierce tissue of the patient's small intestine or stomach wall.

Alternatively or additionally, the anchor may include a self-expanding element that is adapted to be disposed within the patient's small intestine.

Alternatively or additionally, the tether may further include an elongate frictional anchor that is adapted to extend within the patient's small intestine.

Alternatively or additionally, the implantable medical device may further include a dynamic leash that extends from the occlusion device to an anchor site within the patient's stomach.

Alternatively or additionally, the occlusion device may be adapted to extend into the patient's antrum.

Alternatively or additionally, the liner may include a polymeric tube.

Another example may be found in a gastric bypass device. The gastric bypass device includes a funnel device adapted to be disposed within a patient's stomach relative to the patient's pylorus in order to direct stomach contents to flow through the funnel device. A tubular extension is fluidly coupled with the funnel device and extending distally from the funnel device, the tubular extension adapted to extend at through an upper portion of the patient's small intestine in order to prevent the stomach contents flowing through the funnel device and the tubular extension from contacting the upper portion of the patient's small intestine. A tether is secured relative to the funnel device and adapted to limit proximal movement of the funnel device.

Alternatively or additionally, the tether may be adapted to extend within the tubular extension.

Alternatively or additionally, the tether may be adapted to extend outside of the tubular extension.

Alternatively or additionally, the tether may include a proximal end by which the tether is securable relative to the funnel device and a distal end by which the tether is securable to an anchor.

Alternatively or additionally, the gastric bypass device may further include an anchor.

Alternatively or additionally, the anchor may include a self-expanding element that is adapted to be disposed within the patient's small intestine.

Alternatively or additionally, the tether may further include an elongate frictional anchor that is adapted to extend within the patient's small intestine.

Alternatively or additionally, the gastric bypass device may further include a dynamic leash that extends from the funnel device to an anchor site within the patient's stomach.

Another example may be found in a gastric bypass device. The gastric bypass device includes a funnel device adapted to be disposed within a patient's stomach relative to the patient's pylorus in order to direct stomach contents to flow through the funnel device. A tubular extension is fluidly coupled with the funnel device and extending distally from the funnel device, the tubular extension adapted to extend at through an upper portion of the patient's small intestine in order to prevent the stomach contents flowing through the funnel device and the tubular extension from contacting the upper portion of the patient's small intestine. A tether is secured relative to the funnel device and adapted to limit proximal movement of the funnel device. A dynamic leash extends from the funnel device in opposition to the tether.

The above summary of some embodiments, aspects, and/or examples is not intended to describe each embodiment or every implementation of the present disclosure. The figures and the detailed description which follows more particularly exemplify these embodiments.

### Brief Description of the Drawings

The disclosure may be more completely understood in consideration of the following detailed description of various embodiments in connection with the accompanying drawings, in which:
Figure 1 is a schematic view of a portion of a person's gastrointestinal (GI) system;
Figure 2 is a schematic view of an illustrative gastric bypass system disposed within the GI system shown in Figure 1;
Figures 3 through 8 are schematic views of illustrative occlusion devices usable in the illustrative gastric bypass system of Figure 2;
Figures 9A through 9D are schematic views of illustrative tethers usable in the illustrative gastric bypass system of Figure 2;
Figure 10 is a schematic view of a portions of a person's gastrointestinal (GI) system;
Figures 11A through 11C, 12 through 13, and 14A through 14D are schematic views of illustrative tethers usable in the illustrative gastric bypass system of Figure 2 that protect the Papilla of Vater;
Figure 15 is a schematic view of an illustrative gastric bypass system disposed within the GI system shown in Figure 1;
Figure 16 is a schematic view of an illustrative gastric bypass system disposed within the GI system shown in Figure 1; and
Figure 17 is a schematic view of an illustrative gastric bypass system disposed within the GI system shown in Figure 1.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described.

### Detailed Description

The following description should be read with reference to the drawings, which are not necessarily to scale, wherein like reference numerals indicate like elements throughout the several views. The detailed description and drawings are intended to illustrate but not limit the claimed invention. Those skilled in the art will recognize that the various elements described and/or shown may be arranged in various combinations and configurations without departing from the scope of the disclosure. The detailed description and drawings illustrate example embodiments of the claimed invention.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosed invention are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some cases, the term "distal" refers to moving farther into the gastrointestinal system and the term "proximal" refers to moving out of the gastrointestinal system. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

This document relates to devices and methods for the medical treatment of conditions such as obesity and metabolic diseases. For example, this document provides methods and devices for bypassing portions of the GI tract to reduce nutritional update, decrease weight, and/or improve diabetes control.

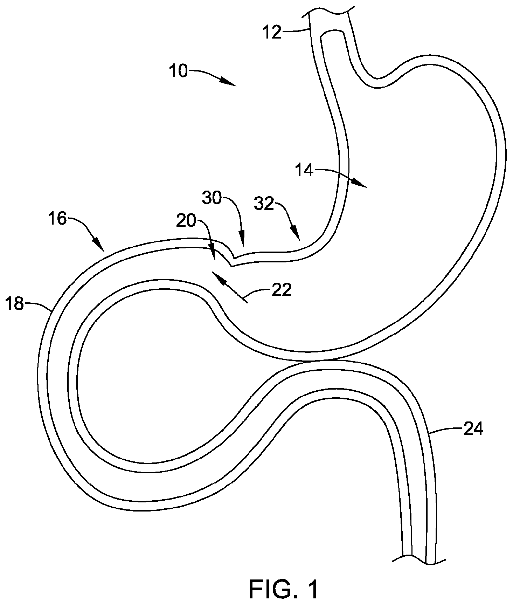

Figure 1 is a schematic view of a portion of a human digestive tract 10. The digestive tract 10 includes an esophagus 12, a stomach 14, and a small intestine 16. The esophagus 12 connects the mouth to the stomach 14 and passes food to the stomach 14. The stomach 14 secretes digestive enzymes and gastric acid to aid in food digestion. The small intestine 16 is the organ where most of the absorption of nutrients and minerals from food takes place. The small intestine 16 includes a duodenum 18, a jejunum 24, and an ileum (not shown ). A pyloric sphincter 20 controls a passage for movement 22 of partially digested food from the stomach 14 into the duodenum 18, which may be about 25-38 centimeters (cm) long. Food then passes to the jejunum 24, which may be about 2.25-2.75 meters (m) long. It will be appreciated that these dimensions are merely illustrative, and may vary from patient to patient. In some cases, the stomach 14 may be considered as including a pylorus 30 that is positioned just upstream of the pyloric sphincter 20. The pylorus 30 may be considered as having a diameter that is greater than that of the pyloric sphincter 20. The stomach 14 may be considered as including an antrum 32 that is positioned just upstream of the pylorus 30. The antrum 32 may be considered as having a diameter that is greater than that of the pylorus 30.

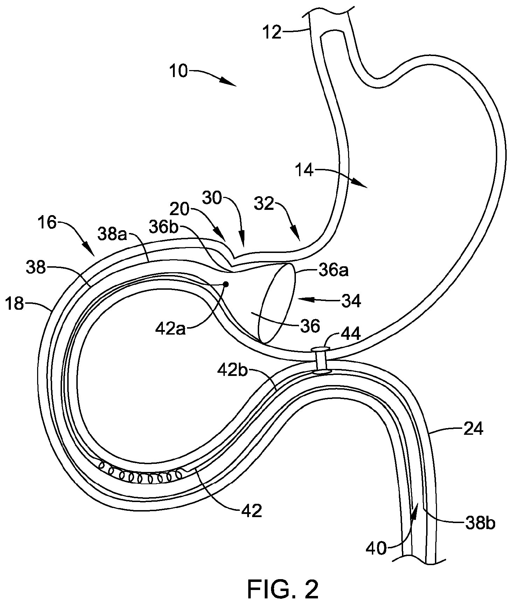

Figure 2 schematically shows an illustrative gastric bypass device 34 shown disposed within the anatomy 10. The gastric bypass device 34 includes an occlusion device 36, which may be adapted to be placed within the pyloric sphincter 20, or within the pylorus 30, or even within the antrum 32, depending on a desired degree of occlusion for the stomach 14. In some cases, the occlusion device 36 may be referred to as a funnel device, for example. The gastric bypass device 34 includes a liner 38 that extends distally from the occlusion device 36. In some cases, the liner 38 may be a separately formed element that is adhesively or otherwise secured to the occlusion device 36. In some cases, the liner 38 may be integrally formed with the occlusion device 36. The liner 38, which in some cases may be referenced as being a tubular extension, may be considered as adapted to allow stomach contents entering the liner 38 to pass through the liner 38. The liner 38 defines a lumen 40 that extends through the liner 38. In some cases, the lumen 40 extends through the liner 38 and is fluidly coupled with an interior of the occlusion device 36. The liner 38 extends from a proximal end 38a, where the liner 38 is coupled to the occlusion device 36, to a distal end 38b.

In some cases, the liner 38 may terminate at a junction between the duodenum 18 and the jejunum 24. In some cases, the liner 38 may end at any desired location along the duodenum 18 or the jejunum 24. In some cases, the liner 38 may terminate at any desired location along the small intestine 16. The liner 38 may be a polymeric sleeve or tube, with no reinforcing members added to it. As a result, the liner 38 may collapse on itself when nothing is passing through the liner 38. In some cases, the liner 38 may include rings or other structure that is adapted to help the liner 38 hold its shape, even when empty. The liner 38 may be selected from a variety of different liner sizes, in order to achieve a desired effect. For example, selecting a smaller diameter liner 38 will slow down stomach emptying, while selecting a larger diameter liner 38 will increase the rate of stomach emptying. The liner 38 may be formed of any suitable material.

Because the occlusion device 36 is a foreign object, the stomach 14 may attempt to push the occlusion device 36 out of the pyloric sphincter 20 and down into the duodenum 18. The stomach 14 may attempt to push the occlusion device 36 out of the pyloric sphincter 20 and back into the stomach 14 itself. A tether 42 extends through the duodenum 18, and is secured at a first end 42a of the tether 42 to the occlusion device 36 and is secured at a second end 42b of the tether 42 to an anchor 44. The anchor 44 may be adapted to be anchored to a side wall of the small intestine 16. In some cases, the anchor 44 may be adapted to be anchored to a side wall of the stomach 14. The anchor 44 may be adapted to pierce into tissue of either the small intestine 16 or the stomach 14. Other versions of the anchor 44 will be described with respect to subsequent Figures. The tether 42 may be adapted to help hold the occlusion device 36 in place at its desired implantation location, against movement caused by the stomach 14 attempting to dislodge the occlusion device 36. In some cases, the anchor 44 can attach to intestine, stomach or even nearby ligaments.

As shown, the occlusion device 36 includes an open proximal mouth 36a and a reduced diameter distal region 36b. Stomach contents within the stomach 14 will pass through the open proximal mouth 36a and be guided towards and through the reduced diameter distal region 36b. From there, the stomach contents will pass into and through the liner 38. By the time that the stomach contents has exited the liner 38 at a distal end 46 thereof, the stomach contents will have bypassed contact with a portion of the small intestine 16, including the duodenum 18 and possible part of the jejunum 24.

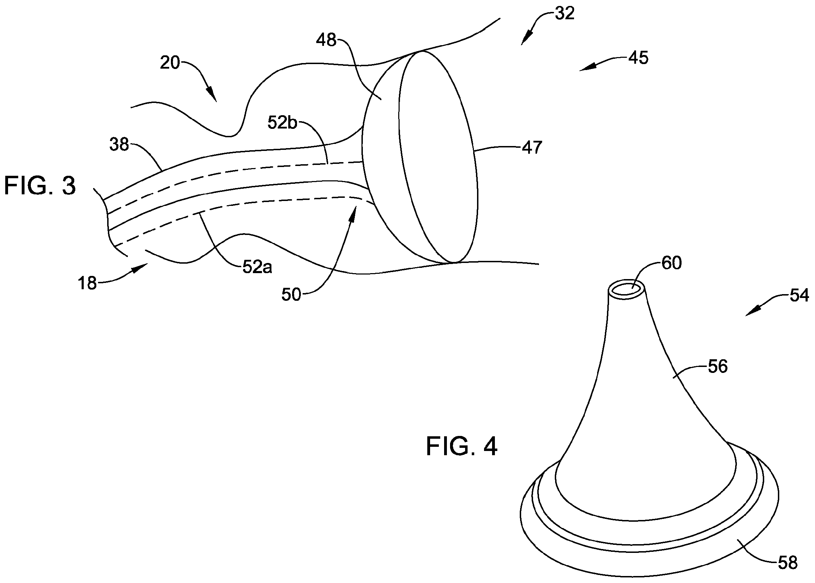

The occlusion device 36 may take a variety of forms. Figure 3 is a schematic view of an illustrative occlusion device 45 shown disposed near the antrum 32. The occlusion device 45 includes an annular ring 47 that is dimensioned to span the anatomy. It will be appreciated that the annular ring 47 may be dimensioned to help locate the occlusion device 45 at a desired position within the anatomy. For example, if there is a desire to locate the occlusion device 45 within the pyloric sphincter 20, the annular ring 47 may have an overall diameter of 1 to 3 cm. If there is a desire to locate the occlusion device 45 within the pylorus 30, the annular ring 47 may have an overall diameter of 2 to 8 cm. If there is a desire to locate the occlusion device 45 within the antrum 32, the annular ring 47 may have an overall diameter of 4 to 12 cm.

The occlusion device 47 includes a tapered body 48 that tapers from the annular ring 47 (which may be considered as defining the maximum outer diameter of the occlusion device 45) to a minimum diameter endpoint 50. In some cases, the liner 38 extends distally from the minimum diameter endpoint 50. In some cases, a tether may be coupled to the tapered body 48. In some cases, the tether may extend outside of the liner 38, as shown for example as a tether 52a. In some cases, the tether may extend inside of the liner 38, either within the lumen 44 or even within a sidewall of the liner 38, as shown for example as a tether 52b.

The tapered body 48 may taper smoothly from its maximum outer diameter to its minimum outer diameter. The tapered body 48 may taper in a step-wise fashion, with one or more abrupt diameter changes. In some cases, the tapered body 48 may have a curved profile. The tapered body 48 may be adapted to allow materials such as food, chyme and other gastric contents to flow through the tapered body 48 while not flowing around an exterior of the tapered body 48. In some cases, the tapered body 48 may be constructed out of an impervious material such as but not limited to a polymeric material. In some cases, the tapered body 48 may include a polymeric membrane disposed over some sort of support frame (not shown).

The thickness, durometer and lubricity of the polymeric material used to form the occlusion device 45 may vary along a length of the occlusion device 44. The occlusion device 45 may have a funnel shape, for example, or a cyclone shape. The occlusion device 45 may have a hemispherical or even a spherical shape. The occlusion device 45 may include an indentation (not shown) to accommodate a support ring. In some cases, the occlusion device 45 may be collapsible in order to aid deliverability.

The occlusion device 45 may be formed of any suitable polymeric or metallic material, as long as that material is adapted for long-term survival in the gastric environment. In some cases, the occlusion device 45 may be formed of silicone or another polymer. The occlusion device 45 may be formed via 3D printing, for example. In some cases, the occlusion device 45 may be molded or even e-spun.

In some cases, the occlusion device 45 may include additional metallic supports (not shown) in order to help provide an outward radial force to better engage the anatomy. In some cases, the material used to form the occlusion device 45 may be thicker near the annular ring 47. The occlusion device 45 may be formed of a shape memory material that allows the occlusion device 45 to have a remembered configuration for deployment, and to be able to temporarily be deformed from the remembered configuration during delivery. While not shown, the occlusion device 45 may include anchors such as outward prongs, hooks, splines or tines. The occlusion device 45 may include a surface treatment that encourages endothelization. These are just examples.

Figure 4 is a schematic view of an illustrative occlusion device 54 that may be considered as being an example of the occlusion device 45. The illustrative occlusion device 54 is formed of a single continuous polymeric body 56 that extends from an annular ring 58 representing a maximum outer diameter of the occlusion device 54 to a minimum diameter endpoint 60. The minimum diameter endpoint 60 may be considered as being adapted to be secured to a tether such as the tether 40. The annular ring 58 may be dimensioned to locate the occlusion device 54 in a desired location relative to the pyloric sphincter 20, the pylorus 30 or the antrum 32, for example.

The occlusion device 54 may be considered as being deformable and endoscopically deliverable. The annular ring 56 is adapted to exert an outward radial force in order to engage the anatomy. If the occlusion device 54 is intended for deployment within the pyloric sphincter 20, the annular ring 58 may have an overall diameter of 1 to 3 cm. If the occlusion device 54 is intended for deployment within the pylorus 30, the annular ring 58 may have an overall diameter of 2 to 8 cm. If the occlusion device 54 is intended for deployment within the antrum 32, the annular ring 58 may have an overall diameter of 4 to 12 cm.

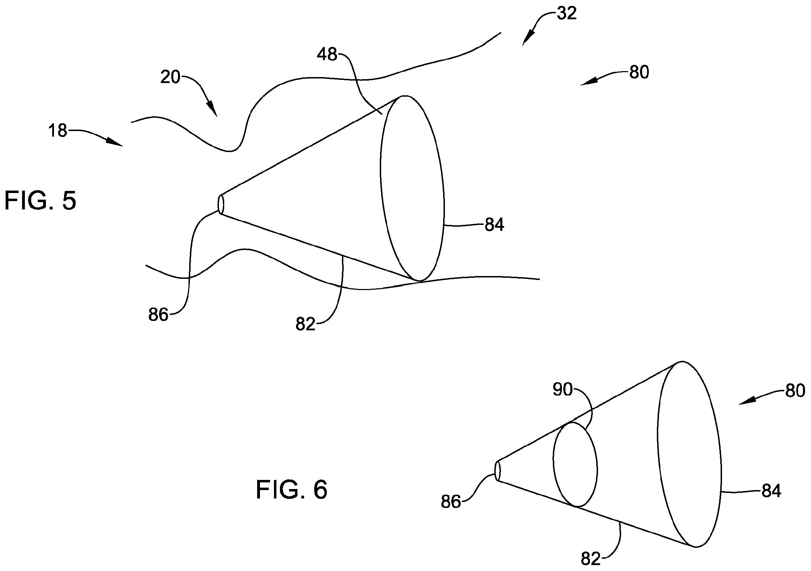

Figure 5 is a schematic view of an illustrative occlusion device 80 that may be considered as being an example of the occlusion device 45. The illustrative occlusion device 80 is shown within the anatomy, and is shown near the antrum 32. In some cases, the occlusion device 80 may occlude from 10 percent to 50 percent of the stomach 14, and may conform to the wall of the stomach 14. The occlusion device 80 includes a thin membrane funnel 82 that is funnel-shaped or conical. The thin membrane funnel 82 may be formed of silicone or expanded polytetrafluoroethylene (e-PTFE), for example. The thin membrane funnel 82 may be formed of a polyurethane that is highly resistant to acids and chemicals. In some cases, a low molecular weight resin such as that available from Cray Valley under the KRASOL^{®} name may be mixed into a polyurethane elastomer that is highly chemically resistant. In some cases, such polybutadiene-urethanes have a rubber character, exceptional resistance to hydrolysis and chemicals, good elasticity and may be reinforced using common rubber fillers.

The occlusion device 80 extends from an annular ring 84 that represents a maximum outer diameter of the occlusion device 80 to a minimum diameter endpoint 86. The minimum diameter endpoint 86 may be considered as being adapted to be secured to the liner 38 (not shown). The minimum diameter endpoint 86 may also be considered as being adapted to be secured to a tether such as the tether 38 (not shown in Figure 5). The annular ring 84, which may be a support ring added to the occlusion device 80, may be dimensioned to locate the occlusion device 80 in a desired location relative to the pyloric sphincter 20, the pylorus 30 or the antrum 32, for example. If the occlusion device 80 is intended for deployment within the pyloric sphincter 20, the annular ring 84 may have an overall diameter of 1 to 3 cm. If the occlusion device 80 is intended for deployment within the pylorus 30, the annular ring 84 may have an overall diameter of 2 to 8 cm. If the occlusion device 80 is intended for deployment within the antrum 32, the annular ring 84 may have an overall diameter of 4 to 12 cm.

The annular ring 84 may be adapted to exert an outward radial force to help hold the occlusion device 80 in position relative to the anatomy. The occlusion device 80 may include partial or entire fiber or metallic reinforcements such as ultra-high weight polyethylene (UHMWPE) or Nitinol. The occlusion device 80 may be manufactured by attaching the thin membrane funnel 82 to the annular ring 84 via sewing, suturing, thermal bonding or chemical bonding, for example.

In some cases, as shown in Figure 6, the occlusion device 80 may include a second, intermediate support ring 90 that helps to support the thin membrane funnel 82. The occlusion device 80 may include a third support ring, a fourth support ring, and so on. The intermediate support ring 90 (and a support ring added to the annular ring 84) may be formed of a shape memory metal such as a nickel-titanium alloy, including Nitinol. While not shown, the occlusion device 80 may include anchors such as outward prongs, hooks, splines or tines. The occlusion device 80 may include a surface treatment that encourages endothelization.

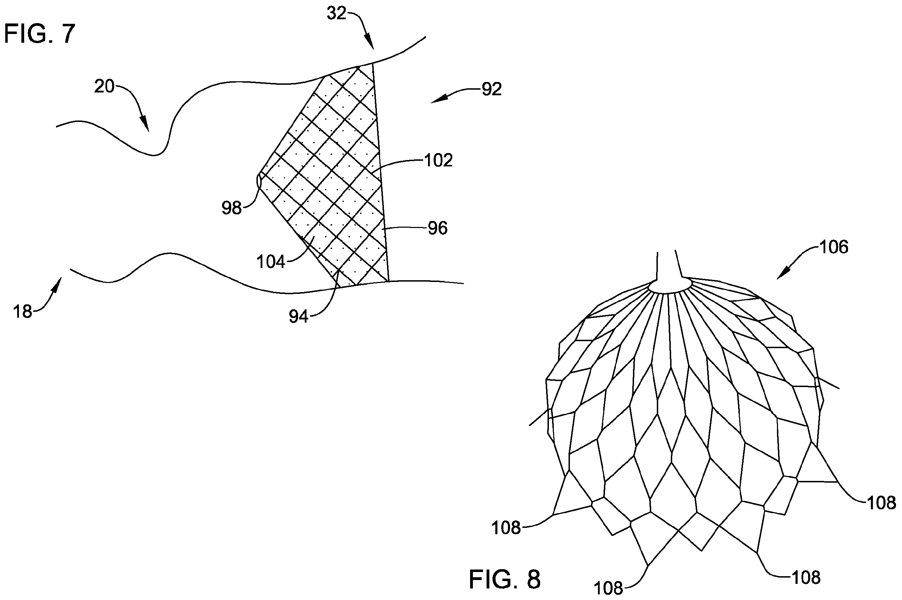

Figure 7 is a schematic view of an illustrative occlusion device 92. The illustrative occlusion device 92 has a structured frame 94 that extends from a maximum diameter opening 96 to a minimum diameter endpoint 98. The minimum diameter endpoint 98 is adapted to be secured to a liner such as the liner 38 and/or a tether such as the tether 42.

The maximum diameter opening 96 may be dimensioned to locate the occlusion device 92 in a desired location relative to the pyloric sphincter 20, the pylorus 30 or the antrum 32, for example. If the occlusion device 92 is intended for deployment within the pyloric sphincter 20, the maximum diameter opening 96 may have an overall diameter of 1 to 3 cm. If the occlusion device 92 is intended for deployment within the pylorus 30, the maximum diameter opening 96 may have an overall diameter of 2 to 8 cm. If the occlusion device 92 is intended for deployment within the antrum 32, the maximum diameter opening 96 may have an overall diameter of 4 to 12 cm.

The structured frame 94 may be woven or braided. In some cases, the structured frame 94 may be a laser cut structure. As shown, the structured frame 94 has a number of individual struts 102 that are connected to provide rigidity to the structured frame 94. The structured frame 94 is adapted to have shape retention such that the structured frame 94 reverts to an expanded configuration (as shown) subsequent to being compressed or otherwise compressed for delivery. The dimensions of the individual struts 102 may be varied to provide particular properties to the structured frame 94. The structured frame 94 may have a cone shape or a funnel shape. The structured frame 94 may be spherical or hemispherical in shape. In some cases, the structured frame 94 may be formed from two or more different parts that are secured together. In some cases, the structured frame 94 may be formed from a laser-cut, expandable tube. The structured frame 94 may be a multi-fiber braided or woven structure. The structured frame 94 may be formed from discrete wires that are soldered, welded or otherwise joined together to form the structured frame 94. The structured frame 94 may be cast from molten metal, for example.

The occlusion device 92 includes a covering or coating 104 (shown in a dotted pattern) that covers at least a portion of the structured frame 94. The covering or coating 104 may be PTFE or e-PTFE. The covering or coating 104 may be silicone or another chemically resistant polymer. The covering or coating 104 may be applied via dip coating, spray coating or e-spinning, for example. While not shown, the occlusion device 92 may include anchors such as outward prongs, hooks, splines or tines. The occlusion device 92 may include a surface treatment that encourages endothelization.

Figure 8 is a schematic view of an illustrative structured frame 106 that may be considered as an example of the structured frame 94. The structured frame 106 includes a number of outward-facing tines 108 that help to anchor the structured frame 106 (and hence the occlusion device including the structured frame 106) in position within the anatomy. When included as part of an occlusion device, the structured frame 106 would include a coating or covering such as the coating or covering 104 shown in Figure 7.

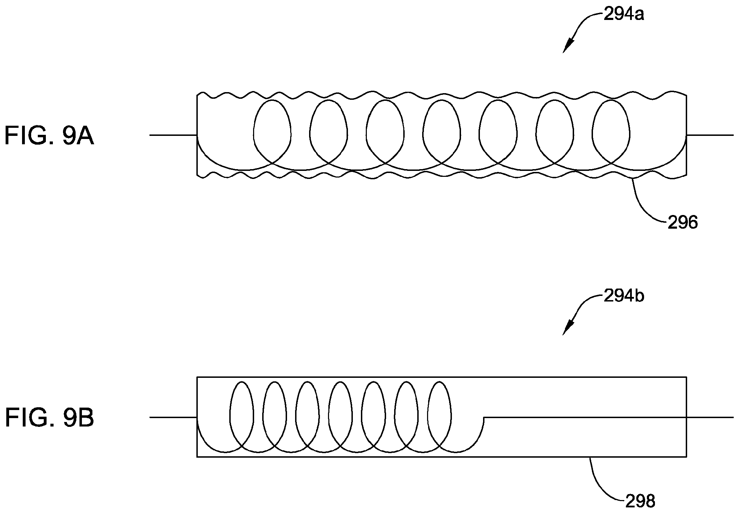

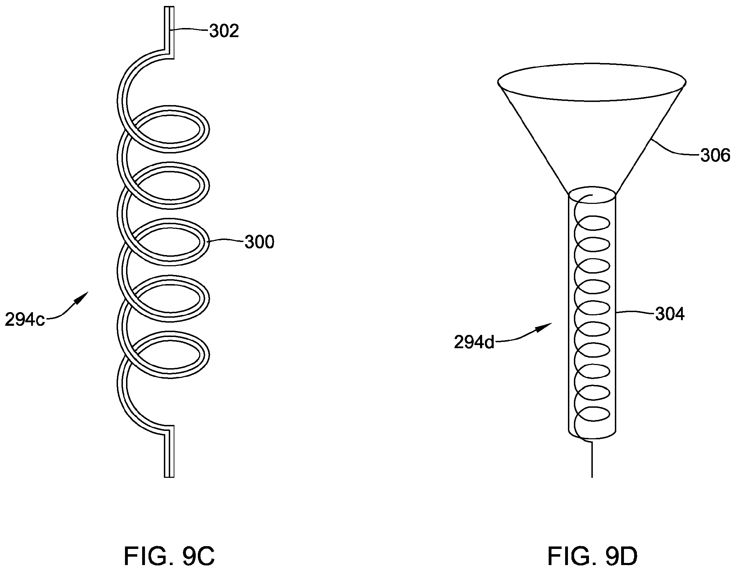

Figures 9 through 14D provide examples of illustrative tethers that may be used as the tether 42 as part of the gastric bypass device 34. In some cases, the tether 42 may simply be a spring that is adapted to provide an increasing return spring force in response to elongation of the spring as gastric motion causes movement of the occlusion device 36. A spring may be formed of any suitable polymeric or metallic material. In some cases, a spring may be formed of Nitinol or stainless steel, for example.

A spring may take a number of forms. In some cases, a spring may have a varying diameter, with a minimum diameter at a midpoint and larger diameters at either end. A spring may have a tapering diameter, from a maximum diameter at one end to a minimum diameter at another end. A spring may have a uniform diameter and pitch from one end to another end. A spring may have a constant outer diameter, but a varying pitch. A spring may have a tapering diameter, from a maximum diameter in the middle to a minimum diameter at either end. These are just examples.

In some cases, the spring or springs may include a covering or coating, particularly if the tether 42 is going to be placed in a position in which the tether 42 may be subjected to fluids and other materials within the gastric system. A covering or coating may reduce friction or other interactions with tissue within the gastric system. A covering or coating may reduce spring interactions with chyme and food, thereby avoiding possibly clogging. A covering or coating may serve as a barrier to the harsh gastric environment. A covering or coating may reduce damage or inflammation at the bile duct and/or at the papilla. In some cases, a covering or coating may be ePTFE, PTFE or other polymers.

Figure 9A shows a spring 294a having a covering 296 that encapsulates the spring 294a and expands and contracts with the spring 294a. Figure 9B shows a spring 294b having a covering 298 that allows the spring 294b to move independently of the covering 298. Figure 9C shows a spring 294c with a covering 300 that is conformal to the filar 302 forming the spring 294c. Figure 9D shows a spring 294d having a covering 304 that is contiguous with a material 306 forming at least part of the occluding device. The springs 294 may be formed of any suitable polymeric or metallic material. In some cases, the spring 294 may be formed of Nitinol or stainless steel, for example.

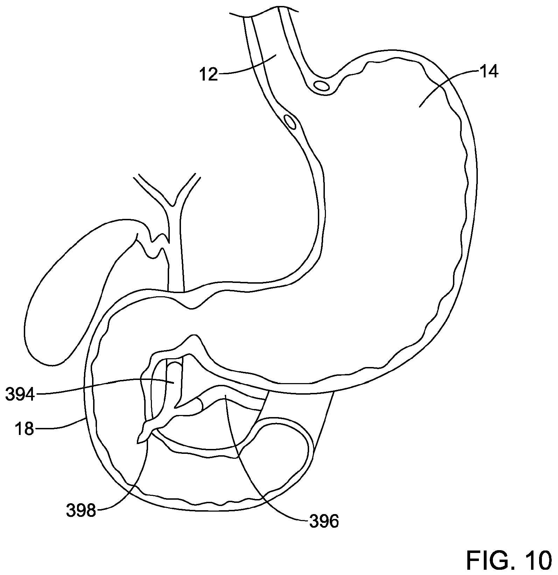

Figure 10 is a schematic view of a portion of the gastrointestinal system that indicates the relative location of a patient's bile duct 394, the patient's pancreatic duct 396 and the patient's Papilla of Vater 398. The Papilla of Vater 398 is where the bile duct 394 and the pancreatic duct 396 are fluidly coupled with the duodenum 18. The Papilla of Vater 398 is located on an inside curve of the duodenum 18, and in some instances may protrude part way into the interior of the duodenum 18. A possible issue with placing a tether in the duodenum 18, particularly if the tether is disposed outside of the liner 38, is that the tether may irritate the Papilla of Vater 398, which can cause inflammation and in turn a variety of possible complications. In some cases, there is a desire to provide tethers that avoid irritating the Papilla of Vater 398.

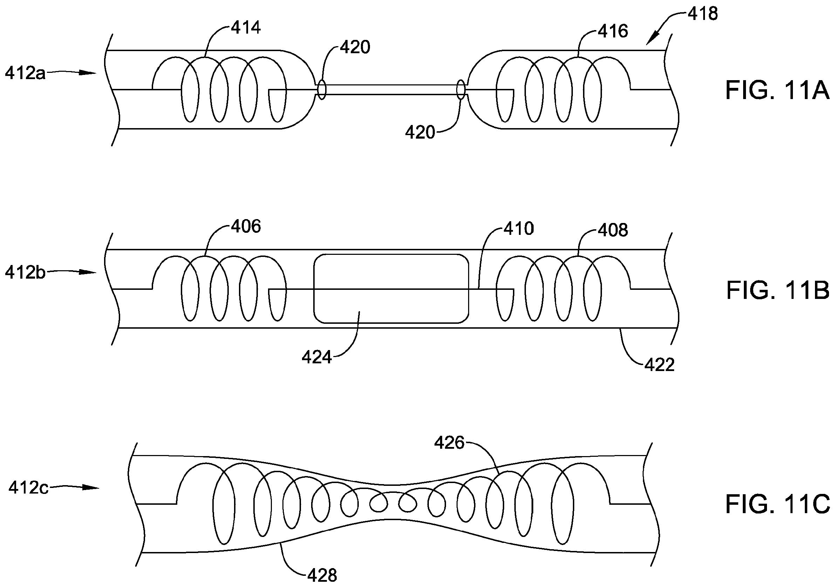

Figures 11A, 11B and 11C provide examples of tethers that are adapted to avoid irritating the Papilla of Vater 398. In Figure 11A, a tether 412a includes a first spring segment 414 and a second spring segment 416, but does not include any metallic structure therebetween. Instead, the tether 412a includes an atraumatic covering 418 that encapsulates the first spring segment 414 and the second spring segment 416. The atraumatic covering 418 is narrowed between the first spring segment 414 and the second spring segment 416 via a pair of sutures 420 that secure the atraumatic covering 418 to an end of the first spring segment 414 and to an end of the second spring segment 416. Thus, nothing metallic will come near the Papilla of Vater 398.

Figure 11B shows a tether 412b that is similar to the tether 400, but includes an atraumatic covering 422 that encapsulates the first spring segment 406, the second spring segment 408 and the member 410 extending therebetween. In some cases, as shown, a soft pillow 424 is disposed between the first spring segment 406 and the second spring segment 408 and is held in place by the atraumatic covering 422. Thus, nothing metallic will come near the Papilla of Vater 398. Figure 11C shows a tether 412c that includes a spring 426 disposed within an atraumatic covering 428. The spring 426 has a varying diameter, with a maximum diameter at either end of the spring 426 and tapering to minimal diameter near a midpoint of the spring 426.

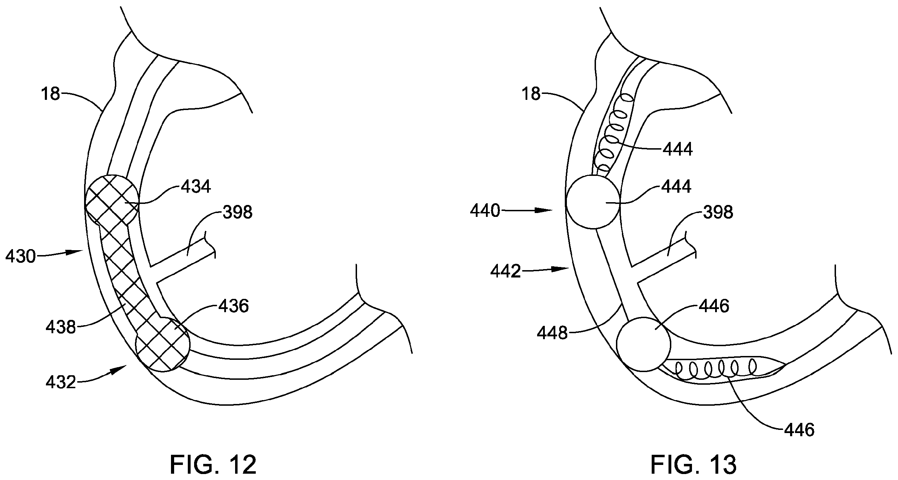

Figure 12 shows a tether 430 that includes a physical standoff 432 disposed over the tether 430, with the tether 430 extending through the physical standoff 432. It will be appreciated that the liner 38 is not shown in this view. In some cases, the physical standoff 432 is able to slide relative to the tether 430. In some cases, the physical standoff 432 is a braided structure formed of a metal such as Nitinol. In some cases, the physical standoff 432 may instead be inflatable, such as an inflatable balloon. As shown, the physical standoff 432 includes a first bulb region 434 and a second bulb region 436, with a narrowed diameter portion 438 extending between the first bulb region 434 and the second bulb region 436. In some cases, the physical standoff 432 may further include additional bulb regions.

Figure 13 shows a tether 440 that includes a physical standoff 442 that forms a part of the tether 440. It will be appreciated that the liner 38 is not shown in this view. The tether 440 includes a first spring segment 444 and a second spring segment 446, with the physical standoff 442 disposed between the first spring segment 444 and the second spring segment 446. The physical standoff 442 may be welded or sewn or bonded to each of the first spring segment 444 and the second spring segment 446, for example. The physical standoff 442 may include a first bulb region 444 and a second bulb region 446, with a stiff member 448 extending between the first bulb region 444 and the second bulb region 446.

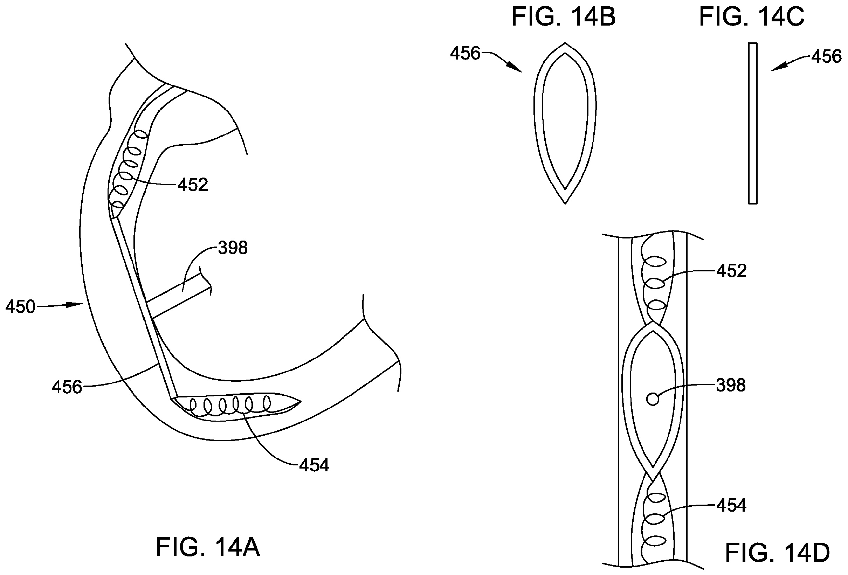

Figure 14A is a schematic view of an illustrative tether 450. It will be appreciated that the liner 38 is not shown in this view. The illustrative tether 450 includes a first spring segment 452 and a second spring segment 454. The tether 450 includes a bow segment 456 that extends between the first spring segment 452 and the second spring segment 454. Figure 14B shows a first view of the bow segment 456 while Figure 14C shows a second view of the bow segment 456. Tension on the tether 450 will rotate the bow segment 456 perpendicular to the Papilla of Vater 398, as shown in Figure 14D.

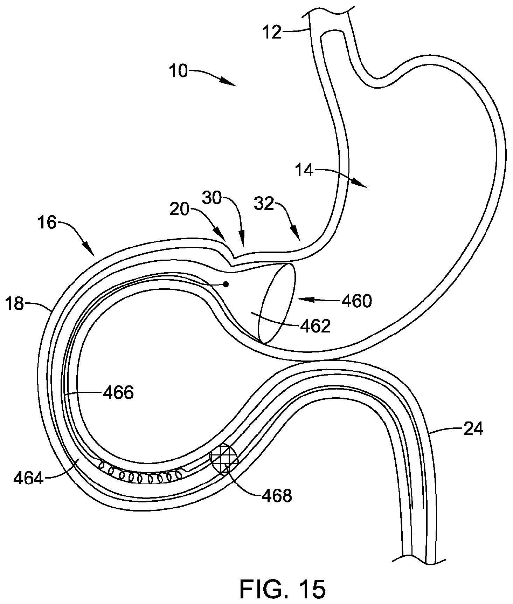

Figure 15 schematically shows an illustrative gastric bypass device 460 shown disposed within the anatomy 10. The gastric bypass device 460 includes an occlusion device 462, which may be adapted to be placed within the pyloric sphincter 20, or within the pylorus 30, or even within the antrum 32, depending on a desired degree of occlusion for the stomach 14. In some cases, the occlusion device 462 may be referred to as a funnel device, for example. The gastric bypass device 460 includes a liner 464 that extends distally from the occlusion device 462. In some cases, the liner 464 may be a separately formed element that is adhesively or otherwise secured to the occlusion device 462. In some cases, the liner 464 may be integrally formed with the occlusion device 462. The liner 464, which in some cases may be referenced as being a tubular extension, may be considered as adapted to allow stomach contents entering the liner 464 to pass through the liner 464. An interior of the liner 464 is fluidly coupled with an interior of the occlusion device 462.

In some cases, the liner 464 may terminate at a junction between the duodenum 18 and the jejunum 24. In some cases, the liner 464 may end at any desired location along the duodenum 18 or the jejunum 24. In some cases, the liner 464 may terminate at any desired location along the small intestine 16. The liner 464 may be a polymeric sleeve or tube, with no reinforcing members added to it. As a result, the liner 464 may collapse on itself when nothing is passing through the liner 464. In some cases, the liner 464 may include rings or other structure that is adapted to help the liner 464 hold its shape, even when empty. The liner 464 may be selected from a variety of different liner sizes, in order to achieve a desired effect. For example, selecting a smaller diameter liner 464 will slow down stomach emptying, while selecting a larger diameter liner 464 will increase the rate of stomach emptying.

A tether 466 extends through the duodenum 18, and is secured at one end to the occlusion device 462 and is secured at a second end to an anchor 468. In some cases, as shown, the anchor 468 may be a self-expanding structure such as a braided structure or a stent structure that is adapted to engage the small intestine 16. The tether 466 may be adapted to help hold the occlusion device 462 in place at its desired implantation location, against movement caused by the stomach 14 attempting to dislodge the occlusion device 462.

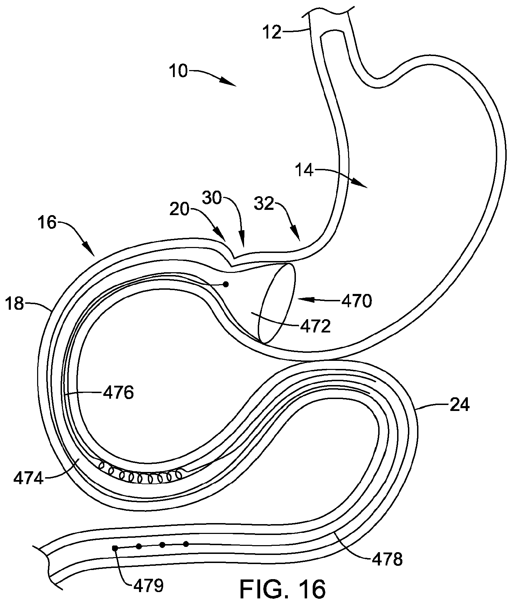

Figure 16 schematically shows an illustrative gastric bypass device 470 shown disposed within the anatomy 10. The gastric bypass device 470 includes an occlusion device 472, which may be adapted to be placed within the pyloric sphincter 20, or within the pylorus 30, or even within the antrum 32, depending on a desired degree of occlusion for the stomach 14. In some cases, the occlusion device 472 may be referred to as a funnel device, for example. The gastric bypass device 470 includes a liner 464 that extends distally from the occlusion device 472. In some cases, the liner 474 may be a separately formed element that is adhesively or otherwise secured to the occlusion device 472. In some cases, the liner 474 may be integrally formed with the occlusion device 472. The liner 474, which in some cases may be referenced as being a tubular extension, may be considered as adapted to allow stomach contents entering the liner 474 to pass through the liner 474. An interior of the liner 474 is fluidly coupled with an interior of the occlusion device 472.

In some cases, the liner 474 may terminate at a junction between the duodenum 18 and the jejunum 24. In some cases, the liner 474 may end at any desired location along the duodenum 18 or the jejunum 24. In some cases, the liner 474 may terminate at any desired location along the small intestine 16. The liner 474 may be a polymeric sleeve or tube, with no reinforcing members added to it. As a result, the liner 474 may collapse on itself when nothing is passing through the liner 474. In some cases, the liner 474 may include rings or other structure that is adapted to help the liner 474 hold its shape, even when empty. The liner 474 may be selected from a variety of different liner sizes, in order to achieve a desired effect. For example, selecting a smaller diameter liner 474 will slow down stomach emptying, while selecting a larger diameter liner 474 will increase the rate of stomach emptying.

A tether 476 extends through the duodenum 18, and is secured at one end to the occlusion device 462 and is secured at a second end to an anchor 478. In some cases, as shown, the anchor 478 may be a frictional feature that extends distally down the small intestine 16. In some cases, the anchor 478 may be long enough such that the natural twists and turns of the small intestine 16 cause the anchor 478 to interact sufficiently with the small intestine 16 to provide an anchoring feature. In some cases, the anchor 478 may include enlarged portions 479 that may provide an additional frictional force between the anchor 478 and the small intestine 16. The tether 476 may be adapted to help hold the occlusion device 472 in place at its desired implantation location, against movement caused by the stomach 14 attempting to dislodge the occlusion device 472.

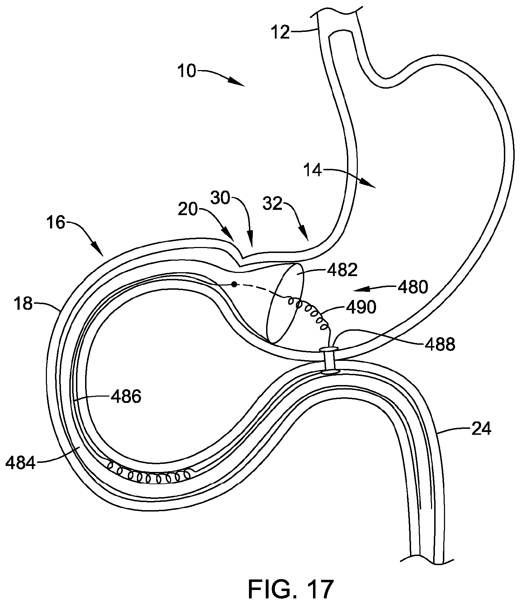

Figure 17 schematically shows an illustrative gastric bypass device 480 shown disposed within the anatomy 10. The gastric bypass device 480 includes an occlusion device 482, which may be adapted to be placed within the pyloric sphincter 20, or within the pylorus 30, or even within the antrum 32, depending on a desired degree of occlusion for the stomach 14. In some cases, the occlusion device 482 may be referred to as a funnel device, for example. The gastric bypass device 480 includes a liner 484 that extends distally from the occlusion device 482. In some cases, the liner 484 may be a separately formed element that is adhesively or otherwise secured to the occlusion device 482. In some cases, the liner 484 may be integrally formed with the occlusion device 482. The liner 484, which in some cases may be referenced as being a tubular extension, may be considered as adapted to allow stomach contents entering the liner 484 to pass through the liner 484. An interior of the liner 484 is fluidly coupled with an interior of the occlusion device 482.

In some cases, the liner 484 may terminate at a junction between the duodenum 18 and the jejunum 24. In some cases, the liner 484 may end at any desired location along the duodenum 18 or the jejunum 24. In some cases, the liner 484 may terminate at any desired location along the small intestine 16. The liner 484 may be a polymeric sleeve or tube, with no reinforcing members added to it. As a result, the liner 484 may collapse on itself when nothing is passing through the liner 484. In some cases, the liner 484 may include rings or other structure that is adapted to help the liner 484 hold its shape, even when empty. The liner 484 may be selected from a variety of different liner sizes, in order to achieve a desired effect. For example, selecting a smaller diameter liner 484 will slow down stomach emptying, while selecting a larger diameter liner 484 will increase the rate of stomach emptying.

A tether 486 extends through the duodenum 18, and is secured at one end to the occlusion device 462 and is secured at a second end to an anchor 488. In some cases, as shown, the anchor 488 may be secured to the tissue of either the small intestine 16 or the stomach 14. In some cases, the anchor 488 may extend through the side wall of the small intestine and the side wall of the stomach 14. The tether 486 may be adapted to help hold the occlusion device 482 in place at its desired implantation location, against movement caused by the stomach 14 attempting to dislodge the occlusion device 482.

In some cases, as shown, a dynamic leash 490 extends between the occlusion device 482 and the anchor 488. In some cases, the dynamic leash 490 may not be secured to the anchor 488, but instead may be secured to an anchor position within a wall of the stomach 14. In some cases, the dynamic leash 490 may be a spring, or may in some way provide a balancing force to that provided by the tether 486. In some cases, the tether 386 may resist proximal movement of the occlusion device 462 while the dynamic leash 490 may resist distal movement of the occlusion device 462 as the anatomy attempts to dislodge the gastric bypass device 480 as a foreign object. The anatomy may view the occlusion device 462 as a large undigested piece of food, and may attempt to force the occlusion device 462 proximally, back up into the stomach, to be further digested.

The materials that can be used for the various components of the medical device systems described herein and the various elements thereof disclosed herein may include those commonly associated with medical devices. In some embodiments, the medical device systems described herein may be made from a metal, metal alloy, polymer (some examples of which are disclosed below), a metal-polymer composite, ceramics, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super-elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super-elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the medical device systems described herein may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the medical device systems. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the medical device systems described herein.

In some embodiments, a degree of Magnetic Resonance Imaging (MRI) compatibility is imparted into the medical device systems described herein. The medical devices described herein may be made of a material that does not substantially distort the image and create substantial artifacts (e.g., gaps in the image). Certain ferromagnetic materials, for example, may not be suitable because they may create artifacts in an MRI image. In some cases, the medical device systems, or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the medical device systems described herein may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), MARLEX^{®} high-density polyethylene, MARLEX^{®}low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the medical device systems described herein and/or other elements disclosed herein may include a fabric material disposed over or within the structure. The fabric material may be composed of a biocompatible material, such a polymeric material or biomaterial, adapted to promote tissue ingrowth. In some embodiments, the fabric material may include a bioabsorbable material. Some examples of suitable fabric materials include, but are not limited to, polyethylene glycol (PEG), nylon, polytetrafluoroethylene (PTFE, ePTFE), a polyolefinic material such as a polyethylene, a polypropylene, polyester, polyurethane, and/or blends or combinations thereof.

It should be understood that this disclosure is, in many respects, only illustrative. Changes may be made in details, particularly in matters of shape, size, and arrangement of steps without exceeding the scope of the invention. This may include, to the extent that it is appropriate, the use of any of the features of one example embodiment being used in other embodiments. The invention's scope is, of course, defined in the language in which the appended claims are expressed.

## Claims

1. An implantable medical device (34) comprising:
an occlusion device (36) adapted to be disposed within a patient's stomach relative to the patient's pylorus in order to prevent stomach contents from flowing around the occlusion device, the occlusion device including a distal outflow end;
a liner (38) extending distally from the occlusion device, the liner defining a lumen (40) extending through the liner, the liner having a proximal end fluidly coupled with the distal outflow end and a distal end adapted to be disposed within the patient's jejunum such that stomach contents entering the occlusion device flow through the liner and exit through the distal end; and
a tether (42) secured relative to the occlusion device and adapted to limit proximal movement of the occlusion device;
**characterized in that** the tether includes a proximal end (42a) by which the tether is securable relative to the occlusion device and a distal end (42b) by which the tether is securable to an anchor (44).

2. The implantable medical device of claim 1 further comprising the anchor, wherein the anchor is adapted to be secured in place relative to the patient's small intestine or stomach wall.

3. The implantable medical device of claim 2, wherein the anchor is adapted to pierce tissue of the patient's small intestine or stomach wall.

4. The implantable medical device of claim 1 further comprising the anchor, wherein the anchor comprises a self-expanding element that is adapted to be disposed within the patient's small intestine.

5. The implantable medical device of claim 1, wherein the tether further comprises an elongate frictional anchor that is adapted to extend within the patient's small intestine.

6. The implantable medical device of any one of claims 1 to 5, further comprising a dynamic leash (490) that extends from the occlusion device to an anchor site within the patient's stomach.

7. The implantable medical device of any one of claims 1 to 6, wherein the occlusion device is adapted to extend into the patient's antrum.

8. The implantable medical device of any one of claims 1 to 7, wherein the liner comprises a polymeric tube.

9. The implantable medical device of any one of claims 1 to 8, wherein:
the occlusion device is a funnel device adapted to be disposed within a patient's stomach relative to the patient's pylorus in order to direct stomach contents to flow through the funnel device; and
the liner is a tubular extension fluidly coupled with the funnel device and extending distally from the funnel device.

## Patentansprüche

1. Implantierbare medizinische Vorrichtung (34), aufweisend:
eine Okklusionsvorrichtung (36), die dazu eingerichtet ist, im Magen eines Patienten relativ zum Pylorus des Patienten angeordnet zu werden, um zu verhindern, dass Mageninhalt um die Okklusionsvorrichtung herum fließt, wobei die Okklusionsvorrichtung ein distales Ausflussende aufweist;
einen sich distal von der Okklusionsvorrichtung erstreckenden Liner (38), wobei der Liner ein Lumen (40) definiert, das sich durch den Liner erstreckt, wobei der Liner ein proximales Ende aufweist, das fluidtechnisch mit dem distalen Ausflussende verbunden ist, und ein distales Ende, das dazu eingerichtet ist, im Jejunum des Patienten angeordnet zu werden, so dass in die Okklusionsvorrichtung eintretender Mageninhalt durch den Liner fließt und über das distale Ende austritt; und
einen Tether (42), der relativ zur Okklusionsvorrichtung gesichert und dazu eingerichtet ist, eine proximale Bewegung der Okklusionsvorrichtung zu begrenzen,
**dadurch gekennzeichnet, dass**
der Tether ein proximales Ende (42a), mit dem der Tether relativ zur Okklusionsvorrichtung befestigbar ist, und ein distales Ende (42b) aufweist, mit dem der Tether an einer Verankerung (44) gesichert werden kann.

2. Implantierbare medizinische Vorrichtung nach Anspruch 1, ferner aufweisend die Verankerung, wobei die Verankerung dazu eingerichtet ist, relativ zur Dünndarm- oder Magenwand des Patienten in Position gesichert zu werden.

3. Implantierbare medizinische Vorrichtung nach Anspruch 2, wobei die Verankerung dazu eingerichtet ist, Gewebe der Dünndarm- oder Magenwand des Patienten zu durchstechen.

4. Implantierbare medizinische Vorrichtung nach Anspruch 1, ferner aufweisend die Verankerung, wobei die Verankerung ein selbstexpandierendes Element aufweist, das dazu eingerichtet ist, innerhalb des Dünndarms des Patienten angeordnet zu werden.

5. Implantierbare medizinische Vorrichtung nach Anspruch 1, wobei der Tether ferner eine längliche Reibungsverankerung aufweist, die dazu eingerichtet ist, sich im Dünndarm des Patienten zu erstrecken.

6. Implantierbare medizinische Vorrichtung nach einem der Ansprüche 1 bis 5, ferner aufweisend eine dynamische Leine (490), die sich von der Okklusionsvorrichtung zu einer Verankerungsstelle im Magen des Patienten erstreckt.

7. Implantierbare medizinische Vorrichtung nach einem der Ansprüche 1 bis 6, wobei die Okklusionsvorrichtung dazu eingerichtet ist, sich in das Antrum des Patienten zu erstrecken.

8. Implantierbare medizinische Vorrichtung nach einem der Ansprüche 1 bis 7, wobei der Liner ein Polymerrohr aufweist.

9. Implantierbare medizinische Vorrichtung nach einem der Ansprüche 1 bis 8, wobei:
die Okklusionsvorrichtung eine Trichtervorrichtung ist, die dazu eingerichtet ist, innerhalb des Magens eines Patienten relativ zum Pylorus des Patienten angeordnet zu werden, um Mageninhalt derart zu lenken, dass er durch die Trichtervorrichtung fließt; und
der Liner eine rohrförmige Verlängerung ist, die fluidtechnisch mit der Trichtervorrichtung verbunden ist und sich distal von der Trichtervorrichtung erstreckt.

## Revendications

1. Dispositif médical implantable (34) comprenant :
un dispositif d'occlusion (36) adapté pour être disposé dans l'estomac d'un patient par rapport au pylore du patient afin d'empêcher que le contenu de l'estomac s'écoule autour du dispositif d'occlusion, le dispositif d'occlusion comprenant une extrémité de sortie distale ;
un revêtement (38) s'étendant de manière distale à partir du dispositif d'occlusion, le revêtement définissant une lumière (40) s'étendant à travers le revêtement, le revêtement ayant une extrémité proximale couplée, de manière fluidique, avec l'extrémité de sortie distale et une extrémité distale adaptée pour être disposée dans le jéjunum du patient de sorte que le contenu de l'estomac entrant dans le dispositif d'occlusion s'écoule à travers le revêtement et sorte par l'extrémité distale ; et
une attache (42) fixée par rapport au dispositif d'occlusion et adaptée pour limiter le mouvement proximal du dispositif d'occlusion ;
**caractérisé en ce que** l'attache comprend une extrémité proximale (42a) grâce à laquelle l'attache peut être fixée par rapport au dispositif d'occlusion et une extrémité distale (42b) grâce à laquelle l'attache peut être fixée à un ancrage (44).

2. Dispositif médical implantable selon la revendication 1, comprenant en outre l'ancrage, dans lequel l'ancrage est adapté pour être fixé en placé par rapport à la paroi de l'intestin grêle ou de l'estomac du patient.

3. Dispositif médical implantable selon la revendication 2, dans lequel l'ancrage est adapté pour percer le tissu de la paroi de l'intestin grêle ou de l'estomac du patient.

4. Dispositif médical implantable selon la revendication 1, comprenant en outre l'ancrage, dans lequel l'ancrage comprend un élément auto-expansible qui est adapté pour être disposé dans l'intestin grêle du patient.

5. Dispositif médical implantable selon la revendication 1, dans lequel l'attache comprend en outre un ancrage à frottement allongé qui est adapté pour s'étendre dans l'intestin grêle du patient.

6. Dispositif médical implantable selon l'une quelconque des revendications 1 à 5, comprenant en outre une attache de sécurité dynamique (490) qui s'étend du dispositif d'occlusion à un site d'ancrage dans l'estomac du patient.

7. Dispositif médical implantable selon l'une quelconque des revendications 1 à 6, dans lequel le dispositif d'occlusion est adapté pour s'étendre dans l'antre du patient.

8. Dispositif médical implantable selon l'une quelconque des revendications 1 à 7, dans lequel le revêtement comprend un tube polymère.

9. Dispositif médical implantable selon l'une quelconque des revendications 1 à 8, dans lequel :
le dispositif d'occlusion est un dispositif d'entonnoir adapté pour être disposé dans l'estomac d'un patient par rapport au pylore du patient afin de diriger le contenu de l'estomac pour qu'il s'écoule à travers le dispositif d'entonnoir ; et
le revêtement est une extension tubulaire couplée, de manière fluidique, avec le dispositif d'entonnoir et s'étendant de manière distale à partir du dispositif d'entonnoir.
